# EUROPEAN PATENT SPECIFICATION

(11) **EP 2 164 401 B1**
(45) Date of publication and mention of the grant of the patent: **26.08.2015**
(21) Application number: 08828134.0
(22) Date of filing: 23.05.2008
(51) Int. Cl.: A61B 8/12, A61B 17/22

(54) **APPARATUS FOR GUIDED CHRONIC TOTAL OCCLUSION PENETRATION**
VORRICHTUNG FÜR GEFÜHRTE PENETRATION BEI CHRONISCHER TOTALER OKKLUSION
APPAREIL DE PÉNÉTRATION ASSISTÉE DANS UNE OCCLUSION TOTALE CHRONIQUE

(30) Priority: 23.05.2007 US 939766 P
(43) Date of publication of application: 24.03.2010
(73) Proprietor: Medinol Ltd., 6158101 Tel Aviv (IL)
(72) Inventor: RICHTER, Jacob, 47226 Ramat Hasharon (IL)
(74) Representative: Kuhnen & Wacker
(86) International application number: PCT/IB2008/003370
(87) International publication number: WO 2009/027846

(56) References cited:
- WO-A-95/09571
- WO-A-2006/117923
- DE-A1- 4 118 714
- US-A1- 2002 029 054
- US-A1- 2002 151 825
- US-A1- 2005 113 688

## Description

### FIELD OF THE INVENTION

The invention relates generally to an apparatus for the guided penetration of a chronic total occlusion (CTO) in a blood vessel and, more particularly, to the use of an ultrasound-based detection system to direct catheter and drill placement during penetration of an occlusion.

### BACKGROUND

The DE 41 18 714 A1 discloses an instrument for braking up the deposit and internal bodily cavity using a piezo-electric crystal attached to a wire threaded through a catheter. The instrument consists of a catheter which has an opening in its wall. A long wire is threaded through the catheter so that its front end projects beyond the inner end of the catheter. The inner end of the catheter is fitted with ultra-sonic generators consisting of piezo-electric crystals which convert high frequency electric currents into ultra sonic waves. An electric cable is connected to the crystal and the cable extends back to through the catheter to a high frequency current generator. The WO 2006/117923 A1 discloses a system for diagnosing and treating the interior of a blood vessel by ultrasonic wave. The system is capable of three dimensionally observing a lesion in a blood flowing blood vessel and concurrently removing thrombus while discriminating in real time an effected part and a vessel, as well as thrombus in flowing blood. The system comprises, at the tip end of a catheter probe, at least a diagnosing ultrasonic probe based on ultrasonic echo, and a treating ultrasonic vibrator capable of removing thrombus in a blood vessel by being vibrated in the longitudinal direction of the probe at its specified frequency including that of ultrasonic wave.

One of the leading causes of human mortality is cardiovascular disease. This commonly begins with stenotic lesions of the coronary arteries, which occur, for example, as a result of the gradual buildup of atheromata, or plaques, along the vessel walls. This buildup leads to a gradual reduction in the diameter of the lumen over time and the subsequent restriction of blood flow. A chronic total occlusion results when a blood vessel becomes completely occluded by plaques for an extended period of time. Such occlusions occur not only in coronary arteries but in other blood vessels as well. A CTO may contain soft plaques, but not infrequently a CTO develops hard plaques which comprise dense, fibrous tissue and calcification at the proximal and distal ends. Until recently, the most common method of treating CTO was bypass surgery, which is a procedure that, unfortunately, involves considerable risk and trauma to the patient.

Advances in modern medicine have led to the development of recanalization procedures for treating such obstructive vascular disorders, for example, balloon angioplasty, atherectomy and stent implantation. These procedures typically require the initial insertion and placement of a guide wire across the occluded region. The guide wire is percutaneously inserted into the blood vessel carrying an interventional catheter, it is directed to and through the stenoses that form the occlusion. The interventional catheter carries, e.g., a balloon and/or stent used to open the occlusion. The primary purpose of the guide wire is to provide an accessible rail over which the physician can route the interventional catheter and subsequently recanalize the occluded lumen using one or more of the aforementioned treatment procedures.

While CTO containing soft plaques are often amenable to penetration with a guide wire, CTO containing hard plaques are often difficult to penetrate successfully with a guide wire, especially where the lesion is heavily calcified. Such situations introduce additional and highly undesirable complexity to the procedure, requiring removal of the guide wire and reinsertion of a stiffer wire. Further, when the tip of the guide wire encounters and fails to penetrate the CTO, it can veer towards the wall of the vessel, thereby possibly damaging, or worse, perforating the vessel wall and other times forming a false lumen.

Some methods have been developed to recanalize this more difficult type of occlusion. In U.S. Patent No. 5,935,108, a needle cannula is mounted within the lumen of a guiding sheath. The needle cannula is used to recanalize the occluded vessel with the help of a guide wire that has an ultrasonic transducer mounted on its distal end to permit the operator to determine whether the occlusion is present or has been successfully crossed, using Doppler shifts in ultrasonic waves. Similarly, U.S. Patent No. 5,938,671 discloses a device that uses a sharpened tip along with two-dimensional ultrasound-based imaging. U.S. Patent Nos. 6,611,458, 6,221,049 and 6,217,527 disclose a method of bypassing a CTO in which a guide wire is redirected through the subintimal space formed between the intimal and adventitial layers of the blood vessel wall. These methods have the disadvantage of only providing a two-dimensional image and of not being able to precisely determine the position of the catheter's distal end relative to the occlusion.

One approach that promotes CTO penetration involves ultrasonic angioplasty. U.S. Patent Nos. 6,482,218 and 5,304,115 describe a wire-shaped ultrasonic catheter that is used to penetrate through hard, calcified deposits of atheroma by pneumatic drilling and through non-calcified material by cavitation. While these patents describe a method of penetrating the CTO, they do not disclose a way to accurately direct the placement of the catheter during the procedure.

The successful penetration of an occlusion in a vessel, particularly a CTO, is heavily dependent upon the ability to monitor, in real time, the position of the distal tip of the guide wire or drilling tip as it is advanced through the lesion. Therefore, there is a need in the art for an apparatus that permits accurate positioning of the guide wire and also facilitates the penetration of a CTO - in coronary blood vessels as well as other types of vessels - and for methods of using such an apparatus. Such an apparatus and method would reduce the risk of penetrating the blood vessel or creating a false lumen.

Accordingly, it is an object of the present invention to provide an apparatus comprising a guide wire having a vibrating end or a tip, in particular a therapeutic tip, that vibrates at a frequency sufficient to penetrate and traverse a vessel occlusion, while simultaneously providing a detectable vibration frequency that permits locating the therapeutic tip, e.g., relative to the body lumen, thereby enabling an operator to direct the guide wire towards a lesion or through an occlusion in a vessel and away from the vessel walls. Another object of the invention is to provide a method of treating a CTO by facilitating penetration of the CTO while simultaneously visualizing the procedure.

### SUMMARY OF THE INVENTION

The invention is laid down in the attached claims, wherein advantageous embodiments thereof are subject-matter of the dependent claims, respectively. The present invention generally relates to an apparatus that facilitates accurate placement of guide wire and drilling tip within the blood vessel during recanalization of a CTO or lesion. The apparatus comprises a detection system that permits differential processing of ultrasonic frequency signals from the guide wire, occlusion and vessel walls using one or more signal receivers that can be oriented at different angles with respect to the area being treated. Preferably, the detection system is an imaging system, more preferably the imaging system is capable of generating real-time three-dimensional images of the CTO or lesion, the blood vessel and the distal tip of the guide wire. Preferably, the apparatus also facilitates penetration of a CTO or other obstructions in a vessel. The method of using the apparatus of the invention facilitates treatment of the occlusion and avoids or reduces the complications and risks associated with treating an occlusion, such as perforation of the walls of the vessel and creation of a false lumen.

The guide wire of the apparatus has a tip, in particular a therapeutic tip, at its distal end, capable of vibrating at user-definable frequencies, including ultrasonic frequencies, that are detectable by the detection or imaging system. The tip or therapeutic tip at the distal end of the guide wire is further capable of vibrating at a frequency sufficient to enable the therapeutic tip of the guide wire to drill through a vessel occlusion. These features may be accomplished by several combinations of particular elements, but are summarized by the non-limiting embodiments described below.

One embodiment relates to a system comprising a drilling component for use in penetrating an occlusion in a body lumen coupled to an imaging component that permits visualization of the drilling procedure resulting in lower risk and fewer complications. The power source and controller work together to energize the transducer causing the therapeutic tip to vibrate or oscillate at a desired frequency. The controller permits operation of the transducer over a range of frequencies and amplitudes, including at frequencies and amplitudes that are useful in drilling and frequencies and amplitudes that are detectable by the receivers. The controller, power source, transducer, therapeutic tip and catheter work together to generate detectable signals from the body lumen and, with the one or more receivers and an imaging system, generate real-time images of the distal end of the guide wire and catheter relative to the walls of the body lumen and the occlusion. This permits the operator to visualize the therapeutic tip and to guide the therapeutic tip and guide wire through the occlusion and away from the vessel walls.

A preselected range of vibrational frequencies generated by the energy-generating system facilitates recanalization of an occluded vessel by energizing an oscillating ceramic motor to a vibrational frequency and amplitude, in particular a vibrational frequency and amplitude sufficient for the therapeutic tip to operate as a drilling device, e.g., to penetrate the occlusion. The oscillating ceramic motor is also made to vibrate at a frequency detectable by the imaging system. In a preferred embodiment, the energy generating system energizes the oscillating ceramic motor to vibrate at an ultrasonic frequency.

### BRIEF DESCRIPTION OF THE DRAWINGS

**Figure 1** depicts an image-based guiding system of the invention.
**Figure 2** depicts an axial cross-section of the distal end of the catheter and guide wire from an embodiment of the system depicted in Fig. 1.
**Figure 3** depicts an axial cross-section of the distal end of the catheter and guide wire from another embodiment of the system depicted in Fig. 1.
**Figure 4a** depicts a transverse section through the catheter of an embodiment of the invention at a point such as **4A** in Fig. 2, showing a plurality of lumens and components therein. **Figure 4b** depicts a transverse section through the catheter of an embodiment of the invention at a point such as **4B** in Fig. 2, showing a cylindrical transducer attached to the catheter and the guide wire positioned in the bore of the transducer.
**Figure 5a** depicts the embodiment of Fig. 2 within a blood vessel having an occlusion; **Figures 5b** and **5c** show portions of an image-based guiding system of the invention in use, at a point of partial penetration of a blood vessel occlusion.

### DETAILED DESCRIPTION

The present invention generally provides a system for use in guiding an apparatus through a body lumen - such as intravascularly - with accuracy, so as to avoid or reduce the risk of perforating the lumen wall or creating a false lumen. The system comprises a flexible, elongated catheter having a proximal and distal end with at least one lumen extending longitudinally therethrough; a guide wire, also having a proximal and distal end and further having a tip, e.g., a therapeutic tip, at its distal end; a vibration means, in particular a transducer, capable of being energized to vibrate at a detectable frequency, which in turn causes the therapeutic tip to vibrate; a power source; a controller for controlling the power source; and a detection system. The detection system comprises one or more receivers and utilizes the controller, which further comprises a processor, for converting signals detected by the receivers into differentiable information. Preferably, the detection system is an ultrasound-based imaging system, comprising an image screen.

The presence of an occlusion in a blood vessel has the deleterious effect of restricting blood flow and affecting the health of the patient. A typical treatment procedure involves percutaneous introduction of a guide wire into a blood vessel and directing the guide wire through the vessel to the occlusion site. According to the present invention, a transducer vibrates the therapeutic tip of the guide wire, enabling the therapeutic tip to penetrate the hard mass of the occlusion. Penetration and traversal of the occlusion may be effected by vibrational drilling and/or cavitation. Also according to the present invention, when the transducer is activated to vibrate at an ultrasonic frequency, one or more receivers capable of ultrasound-based detection of these ultrasonic vibrations can be used to locate and guide the placement of the therapeutic tip. Thus, for example, by energizing an OCM, e.g., on or at the distal end of the guide wire at appropriate frequencies, it is possible not only to drill through the occlusion allowing the guide wire to penetrate the occlusion but also to guide the therapeutic tip in a manner that avoids perforation of the vessel walls or creation of a false lumen using images generated from vibration frequencies detected by one or more external receivers.

The guide wire is slidably mounted on the catheter and its therapeutic tip comprises, or is functionally coupled to, the transducer. The guide wire's total length typically may be greater than that of the catheter such that its proximal end and distal end extend beyond the proximal end and distal end, respectively, of the catheter. In one embodiment, the transducer is preferably a piezoelectric motor, more preferably a miniature oscillating ceramic motor, which is energized from the power source. The energized transducer causes the therapeutic tip of the guide wire to vibrate at a drilling frequency, so that the vibrating therapeutic tip is capable of drilling through the occlusion. The energized transducer also generates a signal of detectable frequency in the therapeutic tip. When the therapeutic tip contacts the surface of an occlusion in the body lumen, it causes the immediate contact area of the occlusion and adjacent tissue, such as walls of the body lumen, to vibrate at a detectable frequency. The power source is functionally connected to the controller, which controls the amount of energy the power source sends to the transducer, and thus the frequency of vibration of the therapeutic tip. In the embodiment in which the detection system is an imaging system, the receivers detect signals produced by the vibrating therapeutic tip, the vibrating occlusion and surrounding tissues and transmit those signals to a processor which generates an image of the therapeutic tip, occlusion and surrounding tissues. The processor may generate real-time images on the image screen. The images may be 3-dimensional. The controller and processor may be connected components or may be the same component, in which case the component may be, in part, a computer - for example, a laptop or desk computer having software to control the power source and to process electrical signals from the receivers into images.

Miniature Oscillating Ceramic Motors (OCM) are known in the art and are disclosed in U.S. Pat. No. 5,453,653 to Zumeris, the specification as it relates to OCMs.

These motors can be made very small and in any shape, and their small size and low energy level requirements make them especially suitable for use inside living organisms. They operate by contacting a surface in an amount sufficient to generate sufficient friction to permit the motor to "crawl" along the contacted surface and change its position relative to the contacted surface when the motor is energized or by vibrating an attached component or the contacted surface. In the present case, for example, an OCM can be made to vibrate the therapeutic tip of the guide wire to facilitate the wire passing through a vessel occlusion. An OCM also can be made to vibrate the therapeutic tip at a detectable frequency. Alternatively, an OCM may vibrate the therapeutic tip at detection frequencies and another vibration transducer may vibrate the therapeutic tip at drilling or cavitation frequencies. OCMs can be adequately insulated to act in aqueous environments. A ceramic motor used in accordance with the present invention may cause the guide wire to "drill" through the calcified or fibrous parts of the occlusion, which are impenetrable by ordinary guide wires. The frequencies utilized in the various embodiments described herein may be varied as specific embodiments may require. A wide range of frequencies, e.g., radio frequency (rf) or ultrasound (us), may be utilized depending upon the type and the location of the tissue being treated and the type and amount of tissue through which the vibrations must pass.

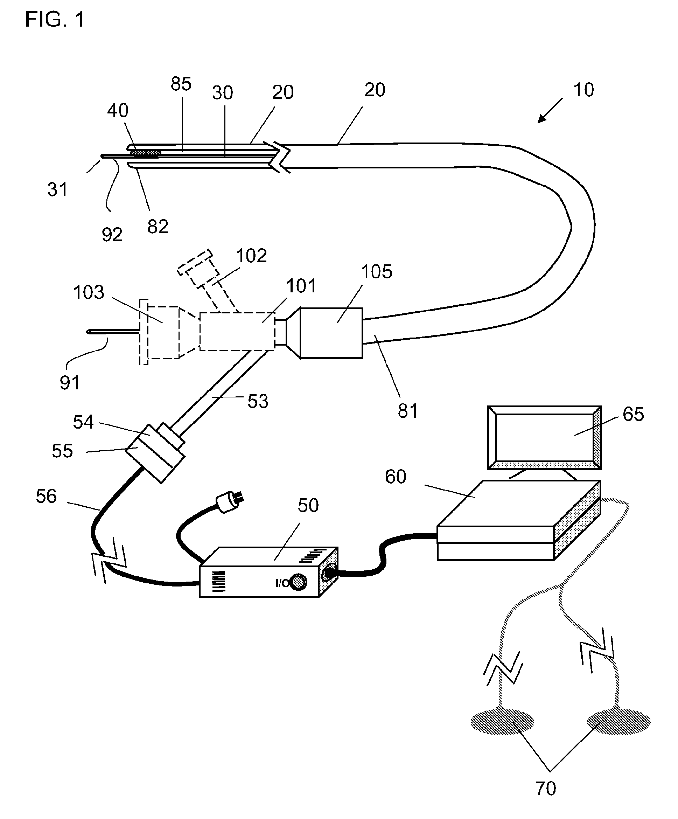

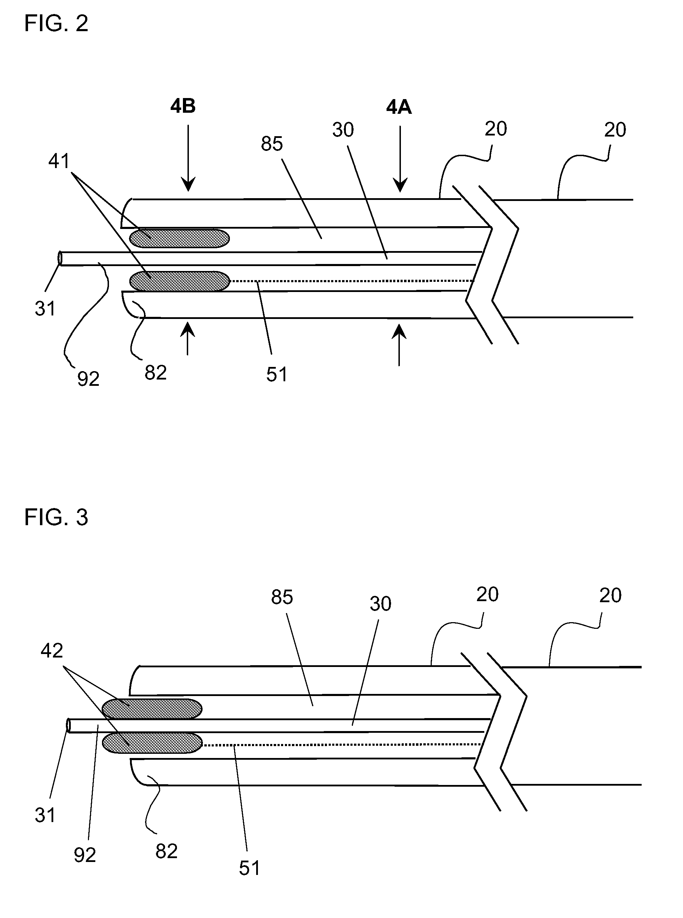

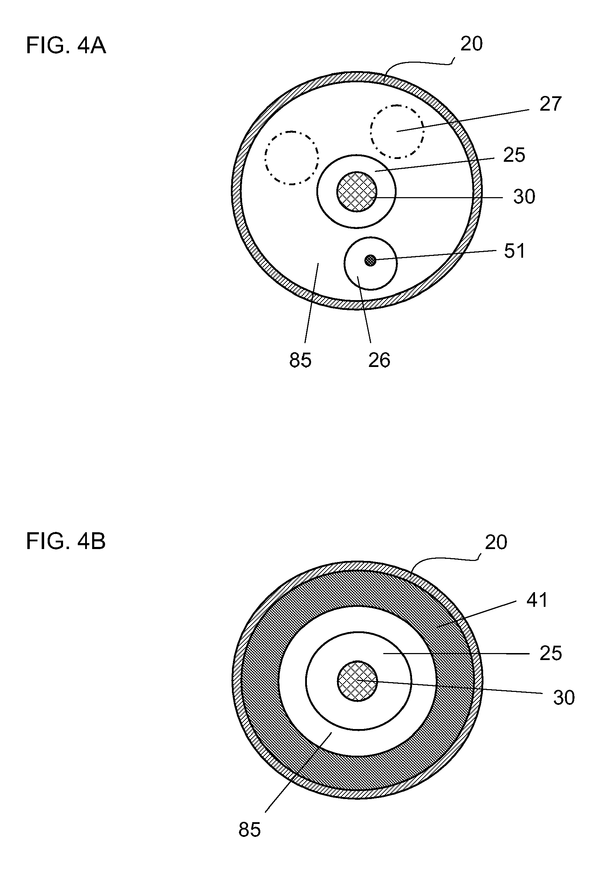

As shown in particular in **FIG. 1****,** a preferred embodiment of the system **10** comprises a catheter **20** having a proximal end **81** and a distal end **82** and a lumenal space **85** therebetween, the lumenal space **85** containing at least one lumen **(see** **Fig. 4a****);** a guide wire **30** within a lumen **25** (not shown in FIG. 1) of the lumenal space **85** of the catheter **20,** the guide wire **30** having a proximal end **91** and a distal end **92** and a therapeutic tip **31** at its distal end **92;** a transducer **40** (embodiments of which are shown in **FIGS. 2** and **3** as **41** and **42,** respectively); a power source **50;** and an imaging system comprising a controller **60;** one or more receivers **70;** and an image screen **65.** The controller **60** processes signals collected by the receivers **70** to create images. Preferably, the controller **60** comprises a computer and software to process, and optionally save, the images. Preferably, the guide wire **30** is longer than the catheter **20,** such that at least its distal end **92** extends beyond the distal end **82** of the catheter **20.** Preferably, the tip of the guide wire **30** is made sufficiently hard and the guide wire itself is sufficiently stiff to penetrate a vessel occlusion (e.g., all commercial guide wires classified as stiff or extra-stiff), yet sufficiently flexible to navigate tortuous blood vessels, such as coronary vessels. The catheter **20** may be any endovascular catheter known in the art, including interventional catheters for delivering endovascular devices, such as stents, or for other therapeutic uses, such as angioplasty.

In addition to processing signals from the receiver **70,** the controller **60** interfaces with the power source **50** and is capable of controlling the amount of energy emitted from said power source **50.** Preferably the power source **50** is a combined sonic and ultrasonic power source such that optimization can be made separately for occlusion penetration frequencies (sonic) and imaging frequencies (ultrasonic) to produce a combined vibration. The controller **60** also interfaces with one or more receivers **70** and the image screen **65.** In one embodiment, the controller **60** is a laptop or desktop computer containing software that controls the power source **50** and processes signals that may be captured by the receivers **70** into a real-time 3-dimensional image on the image screen **65.** The power source **50** generates energy that is transmitted to the transducer **40** and energizes the transducer **40.** Preferably the transducer **40** is a piezo-electric motor; more preferably the piezo-electric motor is an OCM. But, for purposes of generating the image, the source of vibration both for drilling and for imaging may be any other vibrating source.

According to the embodiment depicted in **FIG. 2****,** the transducer **41** is located at the distal end **82** of the catheter **20** and is capable of transferring vibration energy to the distal end **92** of the guide wire **30** near the therapeutic tip **31.** In another embodiment, depicted in **FIG. 3****,** the transducer **42** is located on the therapeutic tip **31** of the guide wire **30.**

When the transducer **40** is a piezoelectric motor or OCM, it is made of a suitable material such as ceramic, quartz or other suitable materials known in the art. The piezoelectric motor is capable of receiving electrical energy and converting it to mechanical energy in the form of longitudinal motion or vibrational wave pulses. The mechanical energy can be used to aid penetration of the occlusion through cavitation and/or pneumatic drilling. The frequency and amplitude of vibration generated by the transducer can be varied by the controller 60 such that parameters suitable for ultrasonic imaging and more effective drilling can be realized. The transduces may be of the same type or of different types. The apparatus comprises two transducers, each energized by the power source 50. One transducer is energized to vibrate the therapeutic tip at a penetration frequency, the other transducer is energized to vibrate the therapeutic tip at an imaging frequency.

The piezoelectric motor may be energized by either an AC or DC source, but preferably it is energized by an AC source. When the piezoelectric motor is an OCM, the frequency of the AC energy input to the OCM will cause the OCM to vibrate in the range of 20-100 kHz, the oscillation depending on the resonant frequency of the material used for the piezoelectric ceramic. When the OCM is energized in a DC pulsed mode, two electrodes are excited by positive voltage and two electrodes are excited by negative voltage. The left side of the energized OCM becomes longer than the right side and the OCM moves to the right, thereby moving the therapeutic tip of the guide wire to the right. When the voltage is stopped, the OCM will move back to its original position. The oscillation (vibrating or pulsating motion) will occur at a frequency dependent on pulse time, preferably 10-50 msec., or a pulsating frequency of 20-100 kHz.

The transducer **40** (and **41, 42**) is capable of receiving energy from the power source **50,** preferably from electrical conducting wires **51** (shown in **FIGS. 2, 3** **and** **4a**) that connect the transducer **40** directly or indirectly to the power source **50.** As depicted in **FIG. 4****,** said electrical conducting wires **51** may lie within a lumen **26** of the lumenal space **85** of the catheter **20,** other than the lumen **25** in which the guide wire **30** lies. The electrical conducting wires **51, 52** extend the entire length of the catheter **20** past its proximal end **81.** In **FIG. 1****,** said electrical conducting wires **51** (not shown) are connected to the power source **50.** For example, electrical conducting wires **51** (see **FIGS. 2, 3** **and** **4a**) exit the catheter **20** at a fitting **105,** which is connected to a hub **101** where the electrical conducting wires **51** connect to a cord **53,** which runs from the hub **101** to a first connector **54.** The first connector **54** is pivotally coupled to a second connector **55;** an electrical cable **56** connects said second connector **55** to the power source **50.** Said cord **53** and said cable **56** contain conductors (not shown) to transmit energy from the power source **50** to the electrical conducting wires **51.**

In an alternative embodiment (not shown), the transducer **40** further comprises a sensor that is capable of receiving energy remotely from a power source and energizing the transducer **40.** In this embodiment, electrical conducting wires **51,** cord **53,** or electrical cable **56** are not required. Specifically, the sensor is adapted to communicate with a power source for selectively generating and transmitting ultrasonic vibrations, such that it receives the transmitted energy and transfers the energy to the transducer, energizing the transducer.

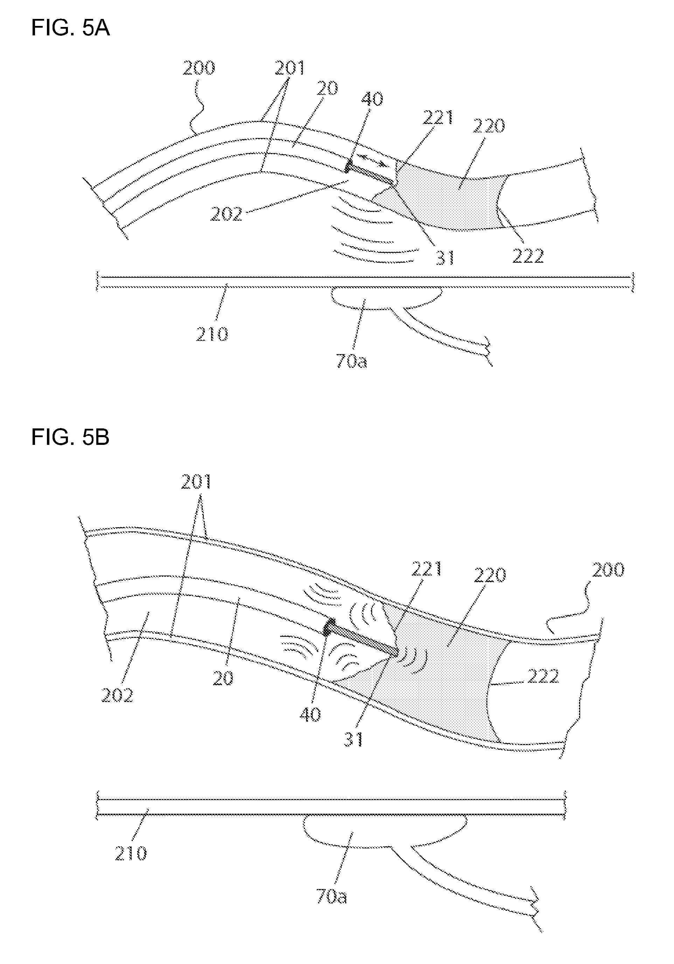

As depicted in **FIG. 5a****,** the catheter **20** and guide wire **30** are introduced into a body lumen, such as a blood vessel **200.** The blood vessel **200** has a vessel wall **201** and a lumen **202,** which lumen **202** is blocked by an occlusion **220.** The occlusion **220** has a proximal cap **221** and a distal cap **222.** The occlusion **220** is expected to comprise fibrous and/or calcified material (not shown), with a higher proportion of calcified material at its proximal and distal caps **221, 222.** The therapeutic tip **31** of the guide wire **30** is placed near the proximal cap **221** of the occlusion **220.** The power source **50** transmits sufficient energy to the transducer **40** to cause the transducer **40** to vibrate the therapeutic tip **31** at a frequency sufficient to penetrate the harder material of the proximal cap **221** of the occlusion **220.** Specifically, the energized transducer causes the therapeutic tip **31** of the guide wire **30** to vibrate, such that - when in a blood vessel **200** having an occlusion **220** and placed in contact with the proximal cap **221** of the blood vessel occlusion **220** - the vibrating therapeutic tip **31** is capable of functioning as a drill to penetrate said occlusion **220.**

During the procedure of penetrating the occlusion **220,** the energized vibrating therapeutic tip **31** is placed at the proximal cap **221** of the occlusion **220** and is made to penetrate the occlusion **220.** The high frequency vibration of the vibrating therapeutic tip **31** permits the therapeutic tip **31** to act like a drill, enabling the distal end **92** of the guide wire **30** to penetrate the occlusion **220,** as depicted in **FIG 5b****.**

In the embodiment depicted in **FIG. 2****,** the catheter-mounted transducer **41** is attached to the distal end **82** of the catheter **20,** but functionally communicates with the therapeutic tip **31** of the guide wire **30.** In this embodiment, the catheter-mounted transducer **41** is cylindrical with a bore through the middle and fits within the lumenal space **85** at the distal end **82** of the catheter **20.** The cylinder bore forms a guide wire lumen, through the center of which the guide wire **30** passes. As shown in **FIGS. 5a-5c****,** after the guide wire **30** and catheter **20** are advanced to the proximal cap **221** of the occlusion **220,** the catheter **20** is secured and the catheter-mounted transducer **41** is energized, causing the catheter-mounted transducer **41** to vibrate the therapeutic tip **31** of the guide wire **30.** In operation, the catheter-mounted transducer **41** is capable of communicating with the therapeutic tip **31** moving the tip in an oscillatory manner that causes the therapeutic tip **31** to vibrate, or move, and function as a drilling device against a lumenal obstruction such as a blood vessel occlusion **220** or CTO. In an alternative arrangement (not shown), the transducer may be one or more slab-shaped transducer(s) disposed on the inner wall of the catheter **20.** The transducer is adapted to frictionally engage the guide wire **30** and move the guide wire **30** relative to the catheter **20.**

In the embodiment depicted in **FIG. 3****,** the guide wire-mounted transducer **42** is attached to the therapeutic tip **31** of the guide wire **30.** In this embodiment, the guide wire-mounted transducer **42** is located at the distal end 82 of the catheter **20** and may be attached to the distal end **82** of the catheter **20,** so as to anchor the guide wire-mounted transducer **42** and therapeutic tip **31** during operation. **Figures 2** and **3** illustrate two non-limiting embodiments of a transducer and its arrangement relative to the catheter and guide wire. Other possible arrangements are within the skill in the art. For example, the transducer **40** may be located more remotely from the therapeutic tip **31,** provided that the catheter **20** is designed to permit vibration for drilling and vibration for detection to be transmitted to the therapeutic tip **31.**

In addition to energizing the transducer **40** to vibrate the therapeutic tip **31** for drilling through the occlusion **220,** the power source **50** provides vibrational energy for detection purposes. Specifically, the power source **50** also transmits energy to the transducer **40** sufficient to generate a vibrational frequency, preferably an ultrasonic or sonic frequency, more preferably an ultrasonic frequency, which frequency is transmitted to the therapeutic tip **31** and adjacent bodily tissues to create detectable signals from the vibrating therapeutic tip **31** and surrounding tissues. These signals may be collected by one or more receivers **70,** as shown for one receiver **70a** in **FIGS. 5a** and **5b****.**

**FIGS. 5a** - **5c** depict an embodiment of the invention in use. The operator places the energized vibrating therapeutic tip **31** in contact with the proximal cap **221** of the occlusion **220,** and the operator maintains contact between the therapeutic tip **31** and some portion of the occlusion **220** throughout the drilling process, as shown in **FIGS. 5a** and **5b****.** When energized to vibrate at a detectable frequency, this contact permits transfer of detectable vibrations to the occlusion **220** and vessel walls **201,** which deflect detectable signals to one or more receivers **70,** as depicted with one receiver **70a** in **FIGS. 5a** and **5b****.** Energizing the transducer **40** also causes the transducer **40** to vibrate the therapeutic tip **31** at a frequency capable of generating detectable signals, which are collected by one or more receivers **70.** The one or more receivers **70** are functionally connected to the controller **60** and image screen **65** (see FIG. 1), such that the signals - in particular from the therapeutic tip, occlusion, vessel walls and surrounding tissues - can be detected by the one or more receivers **70** and may be differentially processed into 3-dimensional images **66** on the image screen **65.**

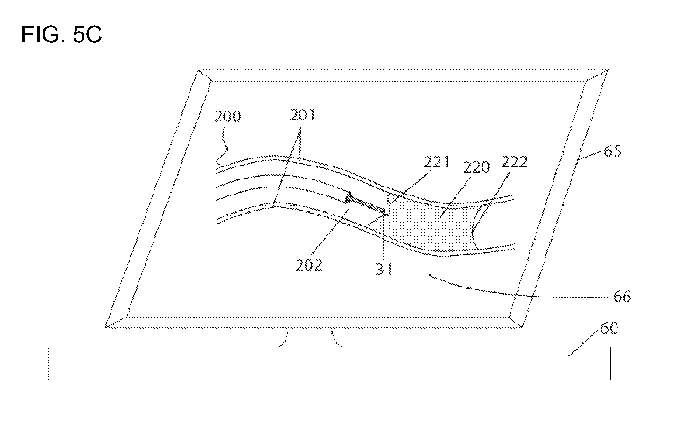

Specifically, the one or more receivers **70,** when placed against the body wall of the patient **210** as exemplified for one receiver **70a** in **FIGS. 5a** and **5b****,** are capable of receiving signals from vibrations of a detectable frequency, preferably a sonic or ultrasonic frequency. The vibrating therapeutic tip **31** produces detectable signals, and transmits vibrations to the occlusion **220** and blood vessel walls **201,** thereby producing detectable signals from those structures. These detectable signals are received by one or more receivers **70** and transmitted to the controller **60,** where they are processed. In this way, the position of the vibrating therapeutic tip **31** relative to the occlusion **220** and blood vessel walls **201** may be visualized on the image screen **65** as 3-dimensional images **66,** as depicted in **FIG. 5c****.**

The system of the invention, as depicted in **FIG. 5c****,** provides real-time feedback in the form of a 3-dimensional image **66** as to the position of the therapeutic tip **31** while drilling into and through the occlusion **220.** This allows the operator to adjust the position of the drilling therapeutic tip **31** and direct the guide wire **30** through the occlusion **220** and away from blood vessel walls **201** to avoid perforating said blood vessel walls **201.**

In an alternative embodiment, the signals - in particular from the therapeutic tip, occlusion, vessel walls and surrounding tissues - are detected by the one or more receivers **70** and may be differentially processed into numerical information that can be transformed into a non-image form perceptible by the operator. Specifically, the detectable signals, which are transmitted to a controller **60** comprising a processor, are processed as information that may be transformed by the controller in any manner known in the art into parameters useful to the operator - such as linear scans, numerical output, audible signals or other parameters. The system of the invention thereby can provide real-time feedback in the form of non-image-based information as to the position of the therapeutic tip **31** while drilling into and through the occlusion **220.** In this way, the position of the vibrating therapeutic tip **31** of the guide wire **30** relative to the occlusion **220** and blood vessel walls **201** may be used by the operator to adjust the position of the drilling therapeutic tip **31** and direct the guide wire **30** through the occlusion **220** and away from blood vessel walls **201** to avoid perforating said blood vessel walls **201.**

Optionally, the hub **101,** shown in **FIG. 1****,** can carry any number of suitable or necessary members useful for intravascular procedures. For example the hub **101** may have a flush port **102** through which a suitable flushing or cooling liquid such as saline solution can be introduced, or an assembly comprising a hemostasis valve **103** through which the guide wire **30** and conducting wires **51, 52** may extend.

Also provided are methods of using the apparatus of the invention for penetrating or recanalizing a vessel occlusion. One such method comprises a) providing a device comprising: i) a guide wire having a proximal end, a distal end and a therapeutic tip at said distal end; ii) a catheter having a proximal end, a distal end, and a longitudinal bore therethrough; iii) a piezoelectric micromotor, said micromotor capable of generating one or more vibrational frequencies when energized by a power source and capable of causing said therapeutic tip to vibrate at said one or more vibrational frequencies; iv) an imaging system comprising one or more receivers for receiving vibrational frequency signals and an imaging screen; b) introducing said guide wire into a blood vessel having vessel walls and an obstruction, and advancing said guide wire until said therapeutic tip of said guide wire contacts said obstruction, wherein said catheter is slidably mounted on said guide wire, said guide wire passing through said longitudinal bore of said catheter; c) advancing said catheter over said guide wire until said distal end of said catheter meets said obstruction, said micromotor now being operatively coupled to said therapeutic tip; d) energizing said piezoelectric micromotor so that said therapeutic tip advances distally through said obstruction in an oscillating or vibrating manner; e) generating detectable vibrational frequency signals from said vibrating therapeutic tip, obstruction and vessel walls via said piezoelectric micromotor; f) detecting said vibrational frequency signals with said one or more receivers of said imaging system, and using said imaging system to generate real-time images of said therapeutic tip relative to said obstruction and said vessel walls; and g) using said generated images to direct said guide wire through said obstruction and away from said vessel walls. The vibration transducer may be a piezoelectric micromotor.

In particular a method for guiding an endovascular device through a blood vessel occlusion is provided. The method comprises a) providing a device, said device comprising: i) a guide wire having a proximal end, a distal end and a therapeutic tip at said distal end; ii) a catheter having a proximal end, a distal end, and a longitudinal bore therethrough; iii) a piezoelectric micromotor, said micromotor capable of generating one or more vibrational frequencies when energized by a power source and capable of causing said therapeutic tip to vibrate at said one or more vibrational frequencies; iv) an imaging system comprising one or more receivers for receiving vibrational frequency signals and an imaging screen; b) introducing said guide wire into a blood vessel having vessel walls and an obstruction, and advancing said guide wire until said therapeutic tip of said guide wire contacts said obstruction, wherein said catheter is slidably mounted on said guide wire, said guide wire passing through said longitudinal bore of said catheter; c) advancing said catheter over said guide wire until said distal end of said catheter is in close proximity of said obstruction, said micromotor now being operatively coupled to said therapeutic tip; d) energizing said piezoelectric micromotor so that said therapeutic tip penetrates said obstruction in an oscillating or vibrating manner; e) generating detectable vibrational frequency signals from said vibrating therapeutic tip, obstruction and vessel walls via said piezoelectric micromotor; f) detecting said vibrational frequency signals with said one or more receivers of said imaging system, and using said imaging system to generate real-time images of said therapeutic tip relative to said obstruction and said vessel walls; and g) using said generated images to direct said guide wire through said obstruction and away from said vessel walls.

In some embodiments, one or more passes of the guide wire through the obstruction may be necessary to clear the blood vessel lumen of obstructive material. To assist penetration of a vessel occlusion by repositioning the guide wire and/or advancing the guide wire and catheter through the occlusion as it is recanalized, the apparatus may further comprise a piezoelectric micromotor designed and placed to permit movement of the catheter relative to the guide wire, as described in U.S. Patent No. 6,238,401.

Such an apparatus may comprise a catheter having a proximal end and a distal end and a longitudinal bore therethrough; a guide wire having a proximal end and a distal end and a therapeutic tip at said distal end; a piezoelectric micromotor; a power source for energizing said piezoelectric micromotor causing said piezoelectric micromotor to vibrate at a first frequency, said piezoelectric micromotor being functionally connected to said therapeutic tip so as to vibrate said therapeutic tip at said first frequency, and for energizing said piezoelectric micromotor at a second frequency, said second frequency being sufficient to create detectable signals; a controller connected to said power source for controlling energy supplied to said piezoelectric micromotor from said power source and thereby control said first frequency; a detection system comprising one or more receivers for collecting said detectable signals and a controller comprising a processor for transforming said signals into differentiable information; wherein said differentiable information may include relative positions of said therapeutic tip, said obstruction and said body lumen, wherein said body lumen has lumen walls, and wherein said detection system permits an operator to use said differentiable information to position said therapeutic tip relative to said body lumen walls and said obstruction.

Methods of using such an apparatus are further provided. One such method comprises advancing said guide wire and said catheter distally by - repeatedly, until said guide wire and said catheter pass substantially through said obstruction - (i) securing said catheter; (ii) releasing said guide wire and energizing said pulling motor so that said guide wire advances distally; (iii) securing said guide wire; (iv) releasing said catheter and energizing said pulling motor so that said pulling motor advances along the guide wire, carrying with it said catheter. Another such method for recanalizing an occlusion, wherein the apparatus comprises a piezoelectric crawling motor capable of pulling said catheter along said guide wire, comprises alternately (i) energizing said crawling motor so that said guide wire advances distally; (ii) energizing said crawling motor so that said guide wire advances proximally; (iii) repeating steps (i) and (ii) a plurality of times until said guide wire has substantially recanalized said obstruction.

In related embodiment, the same piezoelectric micromotor that enables penetration of the occlusion may also move the guide wire and catheter through the occlusion. The method of using such an apparatus comprises a) percutaneously inserting into a body lumen having a target area containing an obstruction an apparatus comprising a cylindrically shaped motor attached to said device, said motor having a longitudinal bore, said motor provided with a motor friction area disposed within said longitudinal bore, a guide wire disposed within said longitudinal bore, said guide wire and said longitudinal bore of said motor sized and adapted to impart friction between said friction area of said motor and said guide wire in an amount sufficient to permit said motor to change position relative to said guide wire by crawling against said guide wire when said motor is energized; b) advancing said guide wire to said target area; c) securing said guide wire; d) energizing said motor so that said motor vibrates and advances along said guide wire to said target area to drill through said obstruction to clear said obstruction from said target area of said lumen; e) vibrating said therapeutic tip, obstruction and walls of said lumen at an ultrasonic frequency, said vibration generating detectable signals; f) collecting said detectable signals and imaging said guide wire, obstruction and walls of said lumen in real time; and g) directing said guide wire through said obstruction and away from said walls of said lumen. In any of the methods of the invention, the catheter may be mounted onto the guide wire after advancing the guide wire to the vessel occlusion, or the catheter may be mounted onto the guide wire prior to advancing the guide wire to the vessel occlusion. In the latter case, the step of advancing the catheter to close proximity of the occlusion may comprise adjusting the position of the catheter on the guide wire relative to the occlusion, such that an operable amount of guide wire extends distal of the distal end of the guide wire.

The image-guided system of the invention may be used to guide the drilling therapeutic tip of the guide wire through the occlusion to widen the passageway sufficiently, for example, to deploy a therapeutic device such as a balloon or stent. Where deployment of a therapeutic device is desired, the catheter 20 may be an interventional catheter carrying a therapeutic device such as an angioplasty balloon or a balloon expandable or self-expanding stent, or the catheter 20 may be exchanged with a second interventional catheter carrying the desired therapeutic device. If such a therapeutic device is to be deployed, the catheter 20 or interventional catheter is advanced through the cleared blood vessel once the guide wire 30 has sufficiently cleared the occlusion 220.

The scope of the invention, is defined by the claims below.

## Claims

1. An intravascular catheter system for penetration and imaging of an occlusion comprising:
a catheter (20) having a proximal end (81) and a distal end (82) and a longitudinal bore (85) therethrough forming a proximal opening and a distal opening;
a guide wire (30) having a proximal end (91)
a distal end (92) with a therapeutic tip (31) ;
a first vibration means for vibrating said therapeutic tip (31) at a first frequency
and connected to said therapeutic tip (31) , said first frequency sufficient to
penetrate an occlusion, said occlusion located in a vessel having walls; a second vibration means for generating a detectable signal at a second
frequency, said second frequency different than said first frequency and
transmitted simultaneously therewith, wherein said detectable signal is
transmitted via the therapeutic tip (31) to the occlusion and the vessel walls
and may be processed into an image of at least one of said tip, occlusion and vessel walls when said therapeutic tip is disposed in said vessel, to enable said tip to be positioned relative to said occlusion and said vessel walls;
a power source (50) for energizing said first and second vibration means to
generate vibrations or oscillations comprising at least one frequency, said power source (50) being connected to a controller (60) for controlling said vibrational frequency and in conjunction with said power source (50)
providing said first and second frequencies to said first and second vibration means simultaneously; and
one or more receivers (70) for collecting said detectable signal simultaneously to the penetration of the occlusion.

2. The system of claim 1, further comprising: an imaging screen (65).

3. The system of claim 2, wherein said controller (60) further comprises a processor for processing said signals into an image on said image screen (65).

4. The system of claim 1, further comprising a processor for transforming said signals into differentiable information of non-image form, said differentiable information including relative positions of said therapeutic tip (31) , said walls, and said obstruction.

5. The intravascular catheter system according to any of claims 1 to 4, wherein the therapeutic tip (31) is a drilling tip.

6. The intravascular catheter system according to claim 5, wherein the drilling tip comprises a rigid material.

7. The intravascular catheter system according to claim 1, wherein said detectable signal has a frequency in the acoustic range selected from the group consisting of ultrasound range, sonic range.

8. The intravascular catheter system according to claim 1, wherein the detectable signal is transmitted at a range of frequencies.

9. The intravascular catheter system according to claim 8, wherein said range of frequencies is selected from the group consisting of ultrasound range, sonic range.

10. The intravascular catheter system of claim 1, wherein said first frequency is in the range selected from the group consisting of ultrasound range, sonic range.

11. The intravascular catheter system of claim 1, wherein said first vibration means is a piezoelectric motor.

12. The intravascular catheter system of claim 1, wherein the second vibration means is a piezoelectric micromotor.

13. The intravascular catheter system of claim 3, wherein said image is a 3-dimensional image (66).

14. The intravascular catheter system of claim 11, wherein said piezoelectric motor is an oscillating ceramic motor.

15. The intravascular catheter system of claim 4, further comprising an imaging screen (65), wherein said processor is capable of generating images from said signals or differentiable information.

## Patentansprüche

1. Intravaskuläres Kathetersystem zur Penetration und Abbildung eines Verschlusses, aufweisend:
einen Katheter (20) mit einem proximalen Ende (81) und einem distalen Ende (82) und einer Längsbohrung (85), wodurch sich eine proximale Öffnung und eine distale Öffnung ergeben;
einen Führungsdraht (30) mit einem proximalen Ende (91) und einem distalen Ende (92) mit einer therapeutischen Spitze (31);
eine erste Vibrationseinrichtung, um die therapeutische Spitze (31) mit einer ersten Frequenz vibrieren zu lassen, wobei die erste Vibrationseinrichtung mit der therapeutischen Spitze (31) verbunden ist, wobei die erste Frequenz geeignet ist,
einen Verschluss zu penetrieren, der sich in einem Gefäß befindet, das Wände aufweist;
eine zweite Vibrationseinrichtung zum Erzeugen eines erfassbaren Signals mit einer zweiten Frequenz, wobei sich die zweite Frequenz von der ersten Frequenz unterscheidet und gleichzeitig mit dieser übertragen wird, wobei das erfassbare Signal über die therapeutische Spitze (31) an den Verschluss und die Gefäßwände übertragen wird und zu einem Bild von zumindest einem von der Spitze, dem Verschluss und der Gefäßwände verarbeitet wird, wenn sich die therapeutische Spitze in dem Gefäß befindet, damit die Spitze im Verhältnis zu dem Verschluss und den Gefäßwänden positioniert werden kann;
eine Leistungsquelle (50) zum Bestromen der ersten und zweiten Vibrationseinrichtungen, um Vibrationen oder Oszillationen zu erzeugen, die zumindest eine Frequenz aufweisen, wobei die Leistungsquelle (50) mit einer Steuerung (60) verbunden ist, um die Vibrationsfrequenz zu steuern und zusammen mit der Leistungsquelle die ersten und zweiten Frequenzen den ersten und zweiten Vibrationseinrichtungen gleichzeitig zur Verfügung zu stellen; und
einen oder mehrere Empfänger (70) zum gleichzeitigen Sammeln des erfassbaren Signals zum Zweck der Penetration des Verschlusses.

2. System nach Anspruch 1, ferner aufweisend: einen Bildschirm (65).

3. System nach Anspruch 2, wobei die Steuerung (60) ferner einen Prozessor aufweist, um die Signale zu einem Bild auf dem Bildschirm (65) zu verarbeiten.

4. System nach Anspruch 1, ferner aufweisend einen Prozessor für das Umwandeln der Signale in differenzierbare Nichtbild-Informationen, wobei die differenzierbaren Informationen relative Positionen der therapeutischen Spitze (31), der Wände und des Verschlusses enthalten.

5. Intravaskuläres Kathetersystem nach einem der Ansprüche 1 bis 4, wobei die therapeutische Spitze (31) eine Bohrspitze ist.

6. Intravaskuläres Kathetersystem nach Anspruch 5, wobei die Bohrspitze ein starres Material aufweist.

7. Intravaskuläres Kathetersystem nach Anspruch 1, wobei das erfassbare Signal eine Frequenz in dem akustischen Bereich aufweist, der aus der Gruppe ausgewählt wird, die aus dem Ultraschallbereich und dem Schallbereich besteht.

8. Intravaskuläres Kathetersystem nach Anspruch 1, wobei das erfassbare Signal in einem Frequenzbereich übertragen wird.

9. Intravaskuläres Kathetersystem nach Anspruch 8, wobei der Frequenzbereich aus der Gruppe ausgewählt wird, die aus dem Ultraschallbereich und dem Schallbereich besteht.

10. Intravaskuläres Kathetersystem nach Anspruch 1, wobei sich die erste Frequenz in dem Bereich befindet, der aus der Gruppe ausgewählt wird, die aus dem Ultraschallbereich und dem Schallbereich besteht.

11. Intravaskuläres Kathetersystem nach Anspruch 1, wobei die erste Vibrationseinrichtung ein piezoelektrischer Motor ist.

12. Intravaskuläres Kathetersystem nach Anspruch 1, wobei die zweite Vibrationseinrichtung ein piezoelektrischer Mikromotor ist.

13. Intravaskuläres Kathetersystem nach Anspruch 3, wobei das Bild ein 3-dimensionales Bild (66) ist.

14. Intravaskuläres Kathetersystem nach Anspruch 11, wobei der piezoelektrische Motor ein oszillierender Keramikmotor ist.

15. Intravaskuläres Kathetersystem nach Anspruch 4, ferner aufweisend einen Bildschirm (65), wobei der Prozessor in der Lage ist, Bilder aus den Signalen oder den differenzierbaren Informationen zu erzeugen.

## Revendications

1. Système de cathéter intravasculaire pour pénétration et imagerie d'une occlusion, comprenant :
un cathéter (20) ayant une extrémité proximale (81) et une extrémité distale (82) et un alésage longitudinal (85) formant à travers une ouverture proximale et une ouverture distale ;
un guide-fil (30) ayant une extrémité proximale (91), une extrémité distale (92) avec une pointe thérapeutique (31) ;
un premier moyen de vibration pour faire vibrer ladite pointe thérapeutique (31) à une première fréquence et connecté à ladite pointe thérapeutique (31), ladite première fréquence étant suffisante pour pénétrer une occlusion, ladite occlusion étant située dans un vaisseau ayant des parois ;
un second moyen de vibration pour générer un signal détectable à une seconde fréquence, ladite seconde fréquence étant différente de ladite première fréquence et étant transmise simultanément avec celle-ci, dans lequel ledit signal détectable est transmis par le biais de la pointe thérapeutique (31) à l'occlusion et aux parois du vaisseau et peut être traité en une image d'au moins un élément parmi ladite pointe, l'occlusion et les parois du vaisseau quand ladite pointe thérapeutique est disposée dans ledit vaisseau, pour permettre à ladite pointe d'être positionnée relativement à ladite occlusion et auxdites parois du vaisseau ;
une source d'alimentation (50) pour alimenter lesdits premier et second moyens de vibration, afin de générer des vibrations ou oscillations comprenant au moins une fréquence, ladite source d'alimentation (50) étant raccordée à un contrôleur (60) pour contrôler ladite fréquence de vibrations et fournir en combinaison avec ladite source d'alimentation lesdites première et seconde fréquences auxdits premier et second moyens de vibration simultanément ; et
un ou plusieurs récepteurs (70) pour collecter ledit signal détectable simultanément à la pénétration de l'occlusion.

2. Système selon la revendication 1, comprenant en outre : un écran d'imagerie (65).

3. Système selon la revendication 2, dans lequel ledit contrôleur (60) comprend en outre un processeur pour traiter lesdits signaux en une image sur ledit écran d'imagerie (65)

4. Système selon la revendication 1, comprenant en outre un processeur pour transformer lesdits signaux en informations différenciables de forme non imagée, lesdites informations différenciables comprenant des positions relatives de ladite pointe thérapeutique (31), desdites parois et de ladite obstruction.

5. Système de cathéter intravasculaire selon l'une quelconque des revendications 1 à 4, dans lequel la pointe thérapeutique (31) est une pointe de perforation.

6. Système de cathéter intravasculaire selon la revendication 5, dans lequel la pointe de perforation comprend un matériau rigide.

7. Système de cathéter intravasculaire selon la revendication 1, dans lequel ledit signal détectable a une fréquence dans la gamme acoustique choisie dans le groupe constitué de la gamme ultrasonore, la gamme audible.

8. Système de cathéter intravasculaire selon la revendication 1, dans lequel le signal détectable est transmis à une gamme de fréquences.

9. Système de cathéter intravasculaire selon la revendication 8, dans lequel ladite gamme de fréquences est choisie dans le groupe constitué de la gamme ultrasonore, la gamme audible.

10. Système de cathéter intravasculaire selon la revendication 1, dans lequel ladite première fréquence est dans la gamme choisie dans le groupe constitué de la gamme ultrasonore, la gamme audible.

11. Système de cathéter intravasculaire selon la revendication 1, dans lequel ledit premier moyen de vibration est un moteur piézoélectrique.

12. Système de cathéter intravasculaire selon la revendication 1, dans lequel le second moyen de vibration est un micromoteur piézoélectrique.

13. Système de cathéter intravasculaire selon la revendication 3, dans lequel ladite image est une image en trois dimensions (66).

14. Système de cathéter intravasculaire selon la revendication 11, dans lequel ledit moteur piézoélectrique est un moteur céramique oscillant.

15. Système de cathéter intravasculaire selon la revendication 4, comprenant en outre un écran d'imagerie (65), dans lequel ledit processeur est capable de générer des images à partir desdits signaux ou informations différenciables.
